# EUROPEAN PATENT APPLICATION

(11) **EP 4 640 233 A1**
(43) Date of publication of application: **29.10.2025**
(21) Application number: 24172294.1
(22) Date of filing: 24.04.2024
(51) Int. Cl.: A61K 38/17, A61K 9/00, A61K 9/06, A61K 45/06, A61K 47/32, A61K 47/38, A61K 47/42, A61P 17/02

(54) **CALRETICULIN HYDROGEL PREPARATIONS FOR ACUTE AND CHRONIC WOUND HEALING**

(71) Applicant: UAB Bioremedium, 01131 Vilnius (LT)
(72) Inventor: Siurkus, Juozas, Vilnius (LT)
(74) Representative: AAA Law

(57) **Abstract**

Stabilized pharmaceutical wound dressing compositions comprising calreticulin protein for use in medical treatment, in particular for topical treatment of acute and chronic wounds, are provided. Pharmaceutical compositions of the present invention comprise calreticulin protein or a functional fragment or derivative thereof and one or more of pharmaceutically acceptable polymeric materials that form hydrogel. More specifically, compositions of present invention can be sprayed onto a wound as a liquid or applied topically in the form of gel. Compositions of the present invention may further comprise serum albumins, preservatives and additional biologically active molecules.

## Description

### TECHNICAL FIELD OF THE INVENTION

The present disclosure relates to stabilized pharmaceutical wound dressing compositions comprising calreticulin protein for use in medicine, in particular for topical treatment of acute and chronic wounds. More specifically, the present invention is directed to hydrogel wound dressings that can be sprayed onto a wound as a liquid or applied topically in the form of gel.

### BACKGROUND OF THE INVENTION

Wound may be described as disruption of anatomical structure and function of an organ, starting from the skin and depending on the injury depth and area might include soft tissues, subcutaneous tissue, muscles, tendons, nerves, vessels, even to the bones. The major mechanisms of injuries are related to mechanical (trauma or surgical interference), chemical, thermal and radiation factors. Based on healing dynamics wounds are sorted in to the two types - acute and chronic.

Normally wound healing or tissue regeneration process is short and dynamic process that goes through several stages: coagulation and hemostasis phase (immediately after injury), inflammation, cell tissue and vascular proliferation, tissue granulation and maturation. Chronic wounds or ulcers have tendency to heal significantly longer compared to acute wounds.

Wound healing is facilitated using wound dressings that perform one or more of the following functions: a) maintain moist environment, b) enhance epidermal migration, c) promote angiogenesis and connective tissue synthesis, d) allow gas exchange between wounded tissue and environment, e) maintain appropriate tissue temperature to improve the blood flow to the wound bed and enhance epidermal migration, f) provide protection against bacterial infection, h) provide debridement action to enhance leucocytes migration and support accumulation of enzymes. Furthermore, it is desirable that wound dressings are non-adherent to the wound and are easily removed after healing. They must be sterile, non-toxic and non-allergenic.

Several wound-dressing technologies are known in the art for use in wound-healing such as traditional dressings, advanced wound dressings for delivery of active substances, antimicrobial dressings, and anti-inflammatory and analgesic dressings.

In traditional dressings, cotton, wool, bandages, and gauzes are mostly used to cover the wound to restore its function underneath. This is still a common approach because of their ease of use, accessibility to most surgical hospitals, and low cost. Such dressings are classified as passive, as they are non-occlusive.

Wound dressings for delivery of active substances are used to transport substances such as antimicrobials, anti-inflammatory agents, and analgesics for wound-healing and are classified as interactive dressings that are semi-occlusive or occlusive. These dressings act as a barrier against penetration of bacteria to the wound environment. In the case of antimicrobial dressings, they are used to kill bacteria or fungi that are present in infected wounds and can reduce the risk of reinfection during wound-healing, surgical procedures, or when changing the dressing. Finally, anti-inflammatory and analgesic dressings can help to reduce the pain and inflammation in wounded areas.

Bioactive wound dressings containing biological agents are also known in the art and may include proteins, such as growth factors, nucleic acids and stem cells, all of which accelerate the regeneration of cells at the site of a wound or adjust the level of substances to be produced when natural wound-healing occurs.

In case of wound dressings for delivery of active substances and bioactive wound dressings, polymeric materials such as hydrogels, hydrocolloids, foams, films, and wafers can be used to transport the substances.

Hydrogels are cross-linked networks of macromolecular compounds characterized by high water absorption capacity. Hydrogels are three-dimensional polymeric networks held together by cross-linked covalent bonds and weak cohesive forces in the form of either hydrogen bonds or ionic bonds. This class of hydrophilic polymeric materials shows an inherent ability to swell in water and other suitable solvents and is capable of imbibing and retaining more than 10% of their weight in water within the gel structure. Hydrogels, together with other chemical compounds, can constitute cosmetic or pharmaceutical formulations, which can find several topical applications on the body and hair surface.

Hydrogels are prepared using a variety of polymeric materials, which can be divided broadly into two categories according to their origin: natural or synthetic polymers. Natural polymers for hydrogel preparation include hyaluronic acid, collagen, chitosan, heparin, alginate, dextran, fibrin.

Synthetic polymers such as poly (vinyl alcohol), polyacrylamide, poly (ethylene oxide), poly (ethylene glycol), sodium polyacrylate, acrylate polymers and copolymers thereof have been used for hydrogel formation. These hydrogels are mainly used in biomedical applications. Natural polymers usually present higher biocompatibility when compared to synthetic polymers. But, on the other hand, synthetic ones are chemically stronger than natural ones. Hydrogels may also be in the form of liquid spray compositions comprising monomers, macromers, and/or polymers that polymerize or otherwise thicken upon or shortly after delivery, to form a hydrogel dressing on a wound as disclosed in WO2003063923A1, US10449272B2.

Examples of synthetic polymers known to be of particular relevance are carbomer, hydroxyethyl cellulose, hydroxypropyl methyl cellulose and xanthan gum, all of which are commercially available from numerous vendors. Carbomer is a term used for a series of polymers primarily made from acrylic acid. Carbomers are white, fluffy powders that are frequently used as gels in cosmetics and personal care products. Hydroxyethyl cellulose and hydroxypropyl methyl cellulose are gelling and thickening agents made chemically through the modification of cellulose. Xanthan gum is an extracellular polymer produced mainly by the bacterium *Xanthomonas campestris,* a polysaccharide with many industrial uses, which can be produced industrially from simple sugars using fermentation process. Traditionally it plays an important role in industrial applications as thickener, emulsion stabilizer and it has been added to water-based drilling fluids due to its pseudoplastic behavior and thermal stability. The structural properties of xanthan in solution can be tuned by the temperature and ionic strength; under high ionic strength or low temperature, xanthan chains are arranged in helical conformation, whereas under low ionic strength or high temperature, xanthan chains are coiled. Xanthan high molecular weight favors the building up of physical and chemical networks, which have been used as carriers for drugs and proteins and as scaffolds for cells. In combination with other polymers xanthan has been applied as excipient in tablets or as supporting hydrogels for drug release applications, particularly due to its acid resistance.

Many biological agents have been tested as bioactive additives to hydrogels for pharmaceutical applications. It is known that exogenous application of growth factors benefits the wound healing process, and this was proven by numerous studies. Among different growth factors, platelet derived growth factor (PDGF) is the most commonly used growth factor which promotes chemotactic recruitment and proliferation of cells and increases angiogenesis. Besides, PDGF, fibroblast growth factor (FGF), epidermal growth factor (EGF), and autologous platelet thrombin are also studied extensively for their application in wound healing process. PDGF and EGF are already approved by FDA for medical use in human applications (as described in Cosmetic Science and Technology, Elsevier, 2017, p. 767-780). However, accumulation of EGF and PDGF is associated with proliferation of certain cancer cells, therefore, it is desirable to provide biologically active proteins that facilitate tissue regeneration.

Enzymatic debridement of necrotic tissues without harming healthy tissue is also a crucial part to promote normal healing process. Ointments comprising papain and collagenase are currently used to digest necrotic tissue. Collagenase acts on the collagen by attacking native collagen and gentle on viable collagen by gradual breakdown of tissue whereas papain attacks cystein residue and associated with inflammatory response. Debridace^{™} is a commercially available wound dressing which increases proteolytic action.

Unexpected effects on the wound healing were observed when testing recombinant human calreticulin protein (CLT) on the chronic diabetic and acute wound healing models. Calreticulin is a highly conserved chaperone protein which has no relationship with the growth factors protein family. In humans the calreticulin protein is a 400 amino acid protein having a molecular weight of 46 kDa. It resides primarily in the endoplasmic reticulum, and is involved in a variety of cellular processes, among them, cell adhesion. Additionally, it functions in protein folding quality control and calcium homeostasis.

In extracellular space calreticulin protein activities are associated with autoimmune regulation, cancer cell inactivation, blood clothing/thrombosis inhibition and facilitation of tissue regeneration.

Calreticulin proteins may be produced as recombinant using bacterial (E. coli) or yeast (Pichia sp.) production systems. Stability and biological activity are the most important quality attributes for recombinant calreticulin protein which depend on the expression host dependent post - translational modifications. It was shown that calreticulin protein produced in Pichia production system using its native signal sequence exhibits biological activity similar to that of native protein most likely because of similar profile of post-translational modifications (Čiplys E, Slibinskas R, Sasnauskas K, Michalak M, Gold LI: Generation of native recombinant secreted human endoplasmic reticulum chaperones by using their native signal sequences in yeast expression systems. PCT patent application WO 2014/011723 A1. Pub. Date: 2014-01-16. Čiplys E, Žitkus E, Gold LI, Daubriac J, Pavlides SC, Højrup P, Houen G, Wang WA, Michalak M, Slibinskas R: High-level secretion of native recombinant human calreticulin in yeast. Microb Cell Fact. 2015 Oct 15;14: 165). Furthermore, it was demonstrated that calreticulin protein exhibits high biological activity in wound healing in various models (Siebert JW, Garg HG, Gold LI. Beneficial wound healing applications of calreticulin and other hyaluronan-associated proteins, US 5,591,716. Pub. date Jan 7, 1997; Gold LI: Therapeutic and cosmetic uses and applications of calreticulin, WO 2011/160082A2. Pub. Date: 2011-12-22).

However, all above described publications in their experiments use aqueous calreticulin protein solutions containing 3.0 mM calcium buffered with either Tris, or PBS that need to be stored either at minus 80° C, or at plus 4° C to maintain proper conformation of this calcium - binding molecule. Such compositions are inconvenient for routine medical treatment of patients both in hospital environments and as over-the-counter preparations.

Therefore it is desirable to provide stabilized compositions comprising calreticulin protein which are convenient for therapeutical use in hospital environments and as over-the-counter preparations.

### SUMMARY OF THE INVENTION

In a first aspect the present disclosure provides stabilized pharmaceutical compositions in the form of hydrogel comprising recombinant human calreticulin protein or a functional fragment or derivative thereof for use in topical treatment of chronic and acute wounds. Stabilized pharmaceutical compositions of the present invention may comprise calreticulin or a functional fragment or derivative thereof in a concentration ranging from about 1 µg/mL to 10 mg/mL, preferably in the range from 5 µg/mL to 1 mg/mL, more preferably in the range between about from 5 µg/mL to 500 µg/mL, most preferably from about 5 µg/mL to 100 µg/mL. The stabilized pharmaceutical composition of the present invention preferably comprises a recombinant calreticulin; preferably wherein the calreticulin is obtained by recombinant expression in yeast cells, such as *Saccharomyces cerevisiae* or *Pichia pastoris.*

Stabilized pharmaceutical compositions of the present invention may be formulated as liquid hydrogel spray, or a gel for administration to body surfaces such as the skin or mucous membranes.

In a second aspect the stabilized pharmaceutical composition of present invention comprises recombinant human calreticulin protein or a functional fragment or derivative thereof and one or more polymeric materials that form hydrogel. Stabilized pharmaceutical compositions of the present invention may comprise polymeric materials such as carbomer, hydroxyethyl cellulose, hydroxypropyl methyl cellulose and xanthan gum, preferably hydroxyethyl cellulose or hydroxypropyl methyl cellulose. In further aspects the stabilized pharmaceutical composition of present invention may be in the form of liquid spray, or a gel. In further aspects the stabilized pharmaceutical composition of present invention may comprise polymeric materials in a concentration ranging between about from 0.5% to 10% w/v of the composition.

In some aspects the stabilized pharmaceutical composition of present invention may further comprise serum albumin proteins, such as bovine serum albumin or human serum albumin. In further aspects the stabilized pharmaceutical composition of present invention may comprise serum albumin proteins in a concentration ranging from about 1 mg /mL to 10 mg /mL.

In some aspects the stabilized pharmaceutical composition of present invention may further comprise preservatives, such as potassium sorbate or sodium benzoate.

In some aspects the stabilized pharmaceutical composition of present invention may further comprise a cytokine, a growth factor, any agonist of wound healing (or effective wound healing agent), including but not limited to small molecule agonists, peptide agonists, chemical agonists, or mixtures thereof. A growth factor according to the present invention can be, for example, platelet-derived growth factor, vascular endothelial growth factor, fibroblast growth factor, epidermal growth factor, TGF-β, and mixtures thereof.

Stabilized pharmaceutical compositions of the present invention may be formulated for topical treatment of chronic and acute wounds by administering such composition topically to a wound of a patient suffering from an acute wound, or from delayed wound healing. Wounds treatable by the pharmaceutical compositions of the present invention include acute wounds, such as originating from thermal and chemical burns, trauma, or surgical interference, and chronic wounds, such as ulcers and diabetic wounds.

### BRIEF DESCRIPTION OF THE DRAWINGS

Fig. 1A. Scratch plate assay (*in vitro* wound healing assay) using adhesive cell lines of human epidermis validation. Calreticulin at a concentration of 100 µg/ml alone (CLT column) or in combination with hydrogen peroxide (CLT/ H₂O₂ column) was used to evaluate its effect on cell migration as compared with scratched cells with no treatment (Control column) and hydrogen peroxide treatment alone (H₂O₂ column). Day 0 - Day 5 lines present time points when cells layers were monitored by microscopy.
Fig. 1B. Scratch plate assay (*in vitro* wound healing assay) on adhesive human epidermis cells using spray hydrogels with calreticulin (prep1-4 columns) and in combination with H₂O₂ (prep1/H₂O₂ - prep4/ H₂O₂ columns).
FIG. 2. Quantitative graph of the spray hydrogel preparations with calreticulin (prep1-4) effect on wound healing. CLT has positive effect on H₂O₂ induced damage, i.e. restores cell ability to cover the scratch area (prep1/H₂O₂).
FIG. 3. CLT activity in CLT solution and CLT spray or gel hydrogel formulations after storage +4 °C.
FIG. 4. CLT activity in CLT solution and CLT spray or gel hydrogel formulations after storage +23 °C.
FIG. 5. CLT activity in CLT solution and CLT spray or gel hydrogel formulations after storage +37 °C.
FIG. 6. In vitro evaluation of regeneration of human epithelial cells, re-formation of the cell layer, CLT spray hydrogel samples.
FIG. 7. In vitro evaluation of regeneration of human epithelial cells, re-formation of the cell layer, CLT gel hydrogel samples.
FIG. 8. Wound area change during healing course when CLT spray and gel hydrogel formulations are used.

### DETAILED DESCRIPTION

The present disclosure relates to the medical use of stabilized pharmaceutical compositions comprising calreticulin in treating acute and chronical wounds of a subject. Similarly, the present disclosure provides a method of treating chronic and acute wounds of a subject, comprising administering to the wound the composition of the present invention. The subject can be any mammal, including a human, non-human primate, or a domesticated mammal such as a cat or a dog. Preferably the subject is a human.

The present invention teaches that calreticulin protein, when formulated into formulations with pharmaceutically acceptable polymeric materials that are used to form hydrogels, exhibit increased stability when stored for prolonged time periods at room temperature (23 °C) and even at 37°C. Also, the functional activity of calreticulin formulations according to the present invention is increased at room temperature down to plus 4° C. Pharmaceutically acceptable means compounds that are useful in preparing a pharmaceutical composition and are generally safe, non-toxic, and neither biologically nor otherwise undesirable and that are acceptable for human pharmaceutical use as well as for veterinary use. Addition of serum albumins to the formulations also contributed to the formulation stability. Serum albumins suitable for use in the compositions of the present invention may be selected from human serum albumin (HSA), or bovine serum albumin (BSA). BSA and HSA are available commercially from several vendors (e.g. Sigma Aldrich) both in native and recombinant forms. Preferably serum albumins for the present inventive compositions are recombinant to conform with pharmacopeia regulations.

Calreticulin is an endoplasmic reticulum protein that is found in a wide range of species and has a highly conserved sequence. In particular, in humans the calreticulin protein is a 400 amino acid protein having a molecular weight of 46 kDa. The term "calreticulin" and "CLT" are used interchangeably herein.

The calreticulin may be described as a mature protein (and not the protein precursor), i.e. one which has undergone post-translational modification and in particular has had the translocation signal removed. In humans the translocation signal is a 17 amino acid hydrophobic N-terminal signal sequence which is cleaved off the 417 amino acid protein precursor. The sequence of human calreticulin precursor (i.e. which includes the 17 amino acid translocation signal) can be found in the UniProt Database at UniProtKB - P27797. The mature human calreticulin protein has SEQ ID NO: 1, which is the sequence of UniProtKB - P27797 minus the 17 amino acid translocation sequence.

In some examples of the present invention the calreticulin may comprise the amino acid sequence of SEQ ID NO: 1 or an amino acid sequence that is at least 85% identical or at least 90% to SEQ ID NO: 1. Preferably the calreticulin comprises the amino acid sequence of SEQ ID NO: 1 or an amino acid sequence that is at least 95% identical to SEQ ID NO: 1. More preferably the calreticulin comprises the amino acid sequence of SEQ ID NO: 1 or an amino acid sequence that is at least 98% or at least 99% identical to SEQ ID NO: 1. In particular, variants of calreticulin are known from homologous sequences from different animals and from non-disease causing polymorphisms already known in the art. Suitable variants can also be made based on single amino acid substitutions, particularly conservative substitutions, that retain the function described herein (for example as determined by the in vitro assay(s) indicated above).

By a "functional fragment" or derivative of a calreticulin is meant that the fragment or derivative is a polypeptide having one or more of the biological properties of calreticulin. Additions, deletions, substitutions and derivatizations of one or more of the nucleotides are contemplated so long as the modifications do not result in loss of functional activity of the fragment or derivative.

The present invention encompasses calreticulin, calreticulin functional fragments and other functional derivatives of calreticulin which have the functional activity of stimulating and promoting healing of a chronic wound or the function of affecting a process associated with enhancing acute wound healing and chronic or impaired wound healing or tissue repair. A functional fragment or a derivative retains at least a portion of the function of calreticulin, such as the activity of promoting chronic wound healing, upregulating TGF-β3 expression in skin, inducing cell migration, stimulating cell proliferation, or binding to a specific anti-calreticulin antibody, which permits its utility in accordance with the present invention. A "fragment" of calreticulin refers to any subset of the molecule, that is, a shorter peptide. A "derivative" or "variant" of calreticulin refers to a molecule substantially similar to either the entire protein or a fragment thereof. Variant/derivative peptides may be conveniently prepared by direct chemical synthesis of the variant peptide or producing the peptide by genetic recombinant technology, using methods well-known in the art.

Accordingly, the present disclosure provides a stabilized pharmaceutical composition comprising calreticulin protein or a functional fragment or derivative thereof and one or more of pharmaceutically acceptable polymeric materials that form hydrogel for use in medical treatment. Therefore, a method for the treatment of medical condition comprising administering said stabilized pharmaceutical composition is provided.

In some embodiments, the present disclosure provides a stabilized pharmaceutical composition for use in medical treatment wherein the pharmaceutical composition is formulated for topical administration and wherein the medical treatment is treatment of chronic and acute wounds.

In some embodiments, the stabilized pharmaceutical composition of the present invention comprises the calreticulin that is human calreticulin. Preferably, preferably the calreticulin has the amino acid sequence of SEQ ID NO: 1 or an amino acid sequence having at least 85% sequence identity to the amino acid sequence of SEQ ID NO: 1. The calreticulin may be obtained from eukaryotic cells, and in particular may be obtained by recombinant expression in either *E.coli* or eukaryotic cells. For example, recombinant calreticulin expressed in E. coli, may be obtained from Sigma Aldrich (# SRP8001, His tagged, >90% (SDS-PAGE)).

Preferably, the calreticulin provided in the stabilized pharmaceutical composition of the present invention is obtained by recombinant expression in yeast cells, such as *Saccharomyces cerevisiae* or *Pichia pastoris.* In particular, the calreticulin may be prepared using recombinant protein production technology based on the secretion of native recombinant protein to the culture medium after expression of human calreticulin precursor including its native signal sequence as described in Čiplys et al, 2014 and 2015, both of which are incorporated herein by reference in their entirety.

In some embodiments, the stabilized pharmaceutical composition of the present invention comprises the calreticulin at a concentration of about from 1 µg/mL to 10 mg/mL, preferably in the range from 5 µg/mL to 1 mg/mL, more preferably in the range between about from 5 µg/mL to 500 µg/mL. In further preferred embodiments, the stabilized pharmaceutical composition of the present invention comprises the calreticulin at a concentration from about 5 µg/mL to 100 µg/mL, more preferably wherein calreticulin is at a concentration of 5 µg/mL, 20 µg/mL or 50 µg/mL. Besides the stability and activity aspect, the low concentrations of calreticulin in the pharmaceutical composition, such as, e.g. 5 µg/mL or 20 µg/mL, are also more financially/commercially attractive.

The stabilized pharmaceutical composition of the present invention is formulated as liquid hydrogel spray or a gel for administration to body surfaces.

In preferred embodiments of the invention, one or more pharmaceutically acceptable polymers are used in the pharmaceutical composition and form hydrogels which provide stabilization functionality. In the preferred embodiments, the stabilized pharmaceutical composition may comprise one or more of the polymeric materials of the group: carbomer, hydroxyethyl cellulose , hydroxypropyl methyl cellulose and xanthan gum. In preferred embodiments the pharmaceutically acceptable polymeric materials are selected from hydroxyethyl cellulose (HEC) and hydroxypropyl methyl cellulose (HPMC).

The stabilized pharmaceutical compositions of the present invention may comprise polymeric materials in a concentration ranging from about 0.5% to 10% w/v of the composition, preferably 0.5% to 5% of the composition. In some embodiments, the stabilized pharmaceutical composition of the present invention comprises a pharmaceutically acceptable polymeric material that is hydroxyethyl cellulose. In further embodiments, HEC is provided at a concentration from 0.1% (w/v) to 4% (w/v), preferably from 0.5% (w/v) to 2% (w/v), more preferably at a concentration of 0.5% (w/v). In some embodiments, the stabilized pharmaceutical composition of the present invention is formulated as liquid hydrogel spray and comprises HEC, for example, at a concentration from 0.1% (w/v) to 4% (w/v), preferably from 0.5% (w/v) to 2% (w/v), more preferably at a concentration of 0.5% (w/v). In some embodiments, the stabilized pharmaceutical composition of the present invention is formulated as hydrogel gel and comprises HEC, for example, at a concentration from 0.1% (w/v) to 4% (w/v), preferably from 0.5% (w/v) to 2% (w/v), more preferably at a concentration of 1.4% (w/v).

In some embodiments, the stabilized pharmaceutical composition of the present invention comprises a pharmaceutically acceptable polymeric material that is hydroxypropyl methyl cellulose. In further embodiments, HPMC is provided at a concentration from 1% (w/v) to 10% (w/v), preferably from 4% (w/v) to 10% (w/v), more preferably at a concentration of 4% (w/v). In some embodiments, the stabilized pharmaceutical composition of the present invention is formulated as liquid hydrogel spray and comprises HPMC, for example, at a concentration from 1% (w/v) to 10% (w/v), preferably from 4% (w/v) to 10% (w/v), more preferably at a concentration of 4.5% (w/v). In some embodiments, the stabilized pharmaceutical composition of the present invention is formulated as hydrogel gel and comprises HPMC, for example, at a concentration from 1% (w/v) to 10% (w/v), preferably from 4% (w/v) to 10% (w/v), more preferably at a concentration of 4% (w/v).

The stabilized pharmaceutical composition of the present invention may further comprise a buffer, for example a phosphate buffer, such as PBS that is diluted 0.1X concentration. The pH of the buffer may be from pH 5 to pH 8, preferably, from 6.5 to 7.2. For example, a buffer may be a phosphate buffer comprising 10mM Na₂HPO₄, 150 NaCl, pH 6.5. In other examples, the pH of the buffer may be 6.8, 7.0 or 7.2, preferably pH is 7.2.

In preferred embodiments, the stabilized pharmaceutical composition may be in the form of liquid spray or a gel and may be stored at temperatures up to 37°C, more preferably stored at room temperature, 22-25°C, for example, at 23°C.

In another embodiment of the invention, the stabilized pharmaceutical composition may further comprise serum albumin proteins, such as bovine serum albumin or human serum albumin. The stabilized pharmaceutical composition of present invention may comprise serum albumin proteins in a concentration ranging from about 1 mg /mL to 10 mg /mL.

In some embodiments, the stabilized pharmaceutical composition may further comprise preservatives, such as potassium sorbate and/or sodium benzoate. In some embodiments, potassium sorbate is provided at concentration from 0.3% to 1%, preferably, at 0.6% (w/v). In some embodiments, sodium benzoate is provided at concentration from 0.3% to 1%, preferably, at 0.5% (w/v).

In yet other embodiments, the stabilized pharmaceutical composition may further comprise a cytokine, a growth factor, any agonist of wound healing (or effective wound healing agent), including but not limited to small molecule agonists, peptide agonists, chemical agonists, or mixtures thereof. A growth factor according to the present invention can be, for example, platelet-derived growth factor, vascular endothelial growth factor, fibroblast growth factor, epidermal growth factor, TGF-β, and mixtures thereof.

Stabilized pharmaceutical compositions of the present invention may be formulated for topical treatment of chronic and acute wounds by administering such composition topically to a wound of a patient suffering from an acute wound, or from delayed wound healing. Wounds treatable by the pharmaceutical compositions of the present invention include acute wounds, such as originating from thermal and chemical burns, trauma, or surgical interference, and chronic wounds, such as ulcers and diabetic wounds.

### Example 1. Wound healing test using spray hydrogels.

In all examples 1 to 5 the calreticulin was a recombinant version that has been produced in Pichia production system according to the methodology as provided in WO2014/011723 A1.

Spray hydrogel preparations used in this Example are presented in Table 1.

**Table 1**

| Code | Calreticulin concentration (µg/mL) | Human Serum Albumin concentration (mg/mL) | Potassium sorbate (% w/v) | Sodium benzoate (% w/v) | Hydroxypropyl Methylcellulose (% w/v) |
|---|---|---|---|---|---|
| Prep1 | 5 | 1 | - | - | 4.5 |
| Prep2 | 20 | 1 | - | - | 4.5 |
| Prep3 | 100 | 1 | - | - | 4.5 |
| Prep4 | 5 | 1 | 0.6 | 0.5 | 4.5 |

PBS (phosphate buffered saline) was added to all preparations to a 0.1X working concentration to maintain pH of the preparations at about 6.5 (10mM Na₂HPO₄, 150 NaCl, pH 6.5).

Wound healing (or scratch) assay was carried out to determine the wound healing abilities of human skin epithelium after the exposure to calreticulin protein alone or in the presence of hydrogen peroxide (H₂O₂), which renders cells unable to cover the scratch. All experiments were carried out at 37°C. Human skin epithelium cells were seeded into 6-well plates and grown until 80-90% confluence. The cell monolayer was scratched with a sterile 1 mL pipette tip and then the preparation containing calreticulin was applied to the scratch surface. Subsequent cell migration and wound area coverage was monitored daily by means of microscopy imaging. The cell-free area was measured by ImageJ software.

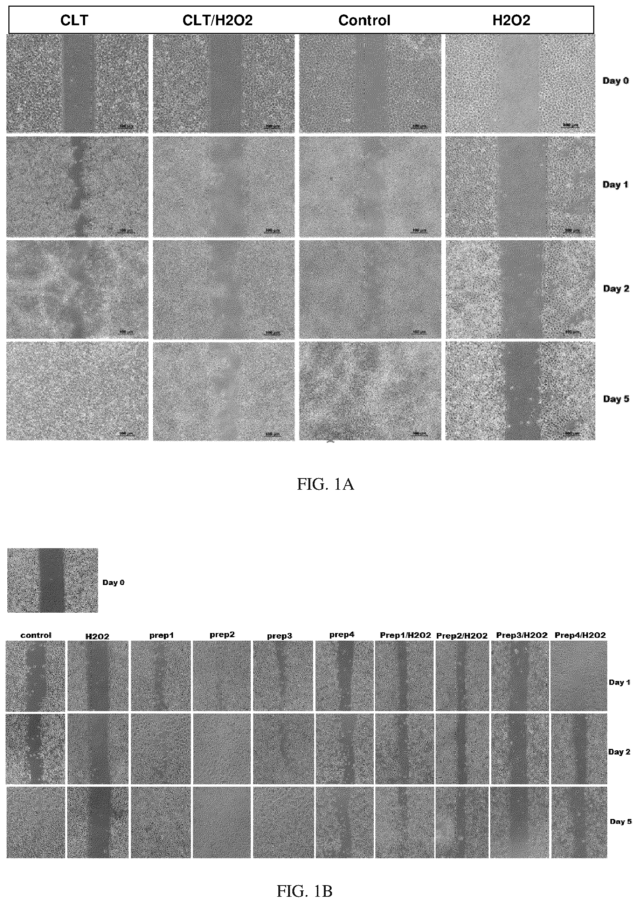

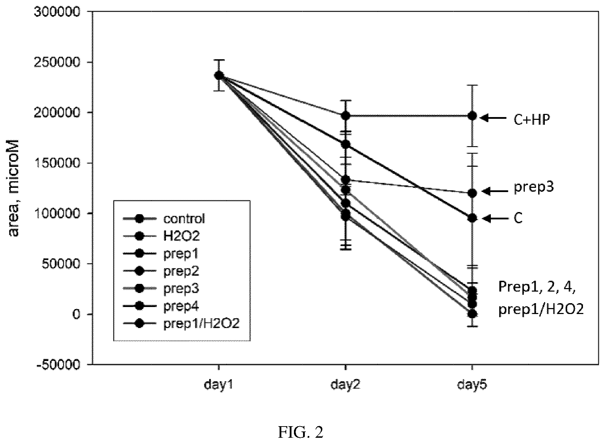

Data presented in Figures 1 and 2 demonstrate that Spray hydrogel preparations comprising calreticulin have positive effect on H₂O₂ - induced damage, i.e. restore cell ability to cover the scratch area.

### Example 2. Wound healing test using hydrogels in the form of gel.

Hydrogel preparations used in this Example are presented in Table 2.

**Table 2**

| Code | Calreticuli n concentrati on (µg/mL) | Human Serum Albumin concentration (mg/mL) | Polymer (% w/v) | Potassium sorbate (% w/v) | Sodium benzoate (% w/v) | PBS (% w/v) |
|---|---|---|---|---|---|---|
| Prep1' | 20 | 1 | Carbomer 0.53% | 0.60 | 0.50 | 98.09 |
| Prep2' | 20 | - | Carbomer 0.53% | 0.60 | 0.50 | 98.09 |
| Prep3' | 20 | 1 | Hydroxyethylcell ulose, 1.40% | 0.60 | 0.50 | 97.50 |
| Prep4' | 20 | - | Hydroxyethylcell ulose, 1.40% | 0.6 | 0.5 | 97.50 |
| Prep5' | 20 | 1 | Xantan gum, 0.84% | 0.6 | 0.5 | 98.06 |
| Prep6' | 20 | - | Xantan gum, 0.84% | 0.6 | 0.5 | 98.06 |

| | | | | | | |
|---|---|---|---|---|---|---|
| pH of all preparations was in the range of 6- 8.5. | | | | | | |

Wound healing (or scratch) assay was carried out in the same way as in Example 1 to determine the wound healing abilities of human skin epithelium after the exposure to hydrogels comprising calreticulin, except that no evaluation of hydrogels effect on H₂O₂ - induced damage was performed. Obtained data showed that all hydrogel preparations comprising calreticulin, or calreticulin in combination with human serum albumin have positive effect on wound healing at 72 hours' time point and were able to restore cell ability to cover the scratch area.

### Example 3. Hydrogel stability results

The composition of hydrogel preparations used in Examples 3 to 5 are provided in Table 3 below. All samples were provided in a buffer: 10 mM Na₂HPO₄, 15 mM NaCl, mM pH 7.2.

| Title* | Form | CLT conc. µg/mL | HSA, mg/ml | HPMC, % (w/v) | HEC, % (w/v) | Potassium sorbate, % (w/v) | Sodium benzoate, % (w/v) | Storage conditions** |
|---|---|---|---|---|---|---|---|---|
| C1 [CLT: 5 µg/mL] | Solution | 5 | 0 | 0 | 0 | 0 | 0 | Stored at -80 °C, or freshy made |
| C2 [CLT:20 µg/mL] | Solution | 20 | 0 | 0 | 0 | 0 | 0 | Stored at -80 °C, or freshy made |
| C3 [CLT: 5 µg/mL] | Solution | 5 | 0 | 0 | 0 | 0 | 0 | Stored at +4, 23 or 37 °C |
| C4 [CLT:20 µg/mL] | Solution | 20 | 0 | 0 | 0 | 0 | 0 | Stored at +4, 23 or 37 °C |
| C-Prep1: (Spray) [CLT:5 µg/mL] | Spray | 5 | 1 | 0 | 0.5 | 0.6 | 0.5 | Freshly made |
| C-Prep1: (Spray) [CLT:20 µg/mL] | Spray | 20 | 1 | 0 | 0.5 | 0.6 | 0.5 | Freshly made |
| C-Prep2:(Gel) [CLT:5 µg/mL] | Gel | 5 | 1 | 4 | 0 | 0.6 | 0.5 | Freshly made |
| C-Prep2:(Gel) [CLT:20 µg/mL] | Gel | 20 | 1 | 4 | 0 | 0.6 | 0.5 | Freshly made |
| Prep1: (Spray) [CLT:5 µg/mL] | Spray | 5 | 1 | 0 | 0.5 | 0.6 | 0.5 | Stored at +4, 23 or 37 °C |
| Prep2:(Gel) [CLT:5 µg/mL] | Gel | 5 | 1 | 4 | 0 | 0.6 | 0.5 | Stored at +4, 23 or 37 °C |
| Prep1: (Spray) [CLT:20 µg/mL] | Spray | 20 | 1 | 0 | 0.5 | 0.6 | 0.5 | Stored at +4, 23 or 37 °C |
| Prep2: (Gel) [CLT:20 µg/mL] | Gel | 20 | 1 | 4 | 0 | 0.6 | 0.5 | Stored at +4, 23 or 37 °C |

| | | | | | | | | |
|---|---|---|---|---|---|---|---|---|
| * - Samples that have "C" in the title are control samples; ** - Storage conditions column denotes what at what conditions the sample were stored prior to appropriate tests. | | | | | | | | |

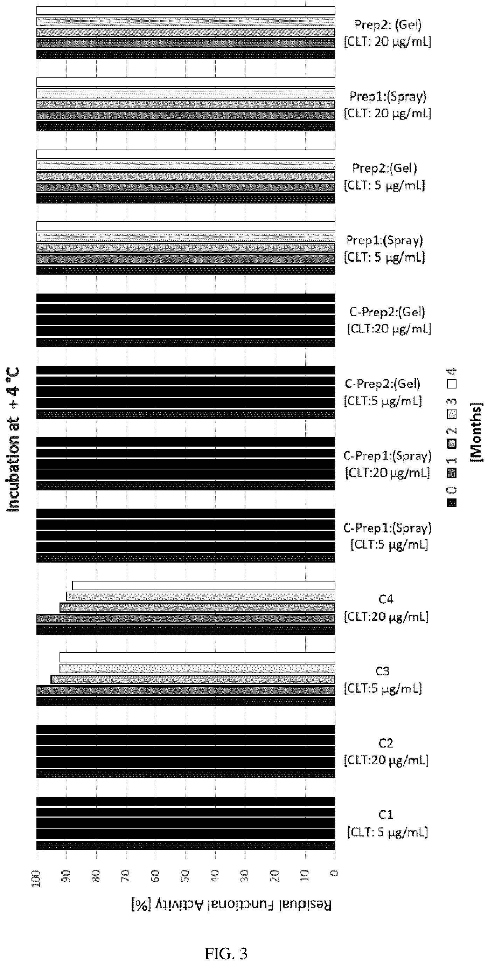

### 3a. Incubation at +4 ° C (Fig. 3)

Accelerated stability studies of the current Prep1 [Spray: containing 5 and 20 µg /mL of CLT] and Prep2 [Gel: containing 5 and 20 µg /mL of CLT] were performed by storage of compositions at +4 °C for 4 months. The functional wound healing in vitro assay was performed byScratch test using human Epidermal keratinocytes HaCAT-KER-CTh (skin) cells. After reaching confluence and forming monolayers, scratch was conducted and cell monolayers were exposed to various concentrations of CLT and CLT based preparations. Effects were assessed after 48 h. The gap of the scratch was evaluated with light microscope, the image was processed with filters and was measured using ImageJ software. The percentage of coverage of scratch area change during the experimental course is provided in Fig. 3 as residual functional activity, i.e. 100% means maximal coverage of scratch area during the set time period.

C1[CLT:5 µg/mL] and C2[CLT:20 µg/mL] - Layers of human Epidermal keratinocytes cells after scratch and constant treatment with pure CLT protein with the concentration 5, 20, µg /mL respectively, which was freshly formulated in phosphate buffer. The evaluation of functional features of fresh CLT was repeated during each stability check point, respectively.

C3[CLT:5 µg/mL] and C4[CLT:20 µg/mL] - Layers of human Epidermal keratinocytes cells after scratch and constant treatment with pure CLT protein with the concentration 5, 20, µg /mL respectively, which was incubated in phosphate buffer at +4 °C for 4 months. The evaluation of CLT preparations were repeated each month, respectively.

C-Prep1:(Spray) [CLT:5 µg/mL] and C-Prep1:(Spray) [CLT: 20 µg/mL] - Layers of human Epidermal keratinocytes cells after scratch and constant treatment with final formulation of Spray containing CLT protein with the concentration 5, 20, µg /mL respectively. The products were freshly formulated before experimental evaluation of scratch layer restoration. The functional evaluation of freshly prepared spray preparations was repeated during whole stability evaluation course during each check point.

C-Prep1:(Gel) [CLT:5 µg/mL] and C-Prep1:(Gel) [CLT: 20 µg/mL] - Layer of human Epidermal keratinocytes cells after scratch and constant treatment with final formulation of Gel containing CLT protein with the concentration 5, 20, µg /mL respectively. The products were freshly formulated before experimental evaluation of scratch layer restoration. The functional evaluation of freshly prepared gel preparations was repeated during whole stability evaluation course during each check point.

Prepl:(Spray) [CLT: 5 µg/mL ] and Prepl:(Spray) [CLT: 20 µg/mL ] - Layer of human Epidermal keratinocytes cells after scratch and constant treatment with final formulation of Spray containing CLT protein with the concentration 5, 20, µg /mL respectively, which was incubated at +4 °C for 4 months. The functional features of products were experimentally evaluated using scratch layer restoration assay, after incubation of formulations during whole stability evaluation course every month, up to 4 months.

Prep1:(Gel) [CLT: 5 µg/mL ] and Prep1:(Gel) [CLT: 20 µg/mL ] - Layer of human Epidermal keratinocytes cells after scratch and constant treatment with final formulation of Gel containing CLT protein with the concentration 5, 20, µg /mL respectively, which was incubated at +4 °C for 4 months. The functional features of products were experimentallly evaluated using scratch layer restoration assay, after incubation of formulations during whole stability evaluation course every month, up to 4 months.

As can be seen from the results in Fig. 3, while the control samples that were stored for up to 4 months (C3 and C4 control samples) started to show loss of CLT activity after 2 months of storage at +4 °C, in cases where CLT was stored in the Spray or Gel formulations (Prepl:(Spray) and Prep2:(Gel) samples) for up to 4 months, the activity of CLT remained the same as before storage of the formulation.

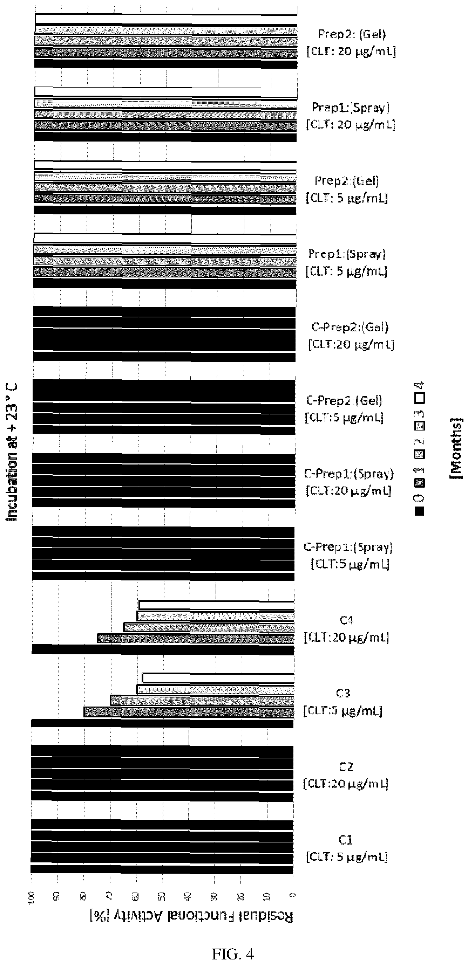

### 3b. Incubation at +23 ° C (FIG. 4)

The samples were formulated and stored using the same principle as in 3a experiment, except that the storage of up to 4 months was performed at +23 ° C temperature. The scratch assay and respective calculations for presentation of the results were performed and presented in the same manner as the assays in Example 3a. The results are provided in FIG. 4; SD values were ranging within the range of 5-15%.

As can be seen from the results in Fig. 4, while the control samples that were stored for up to 4 months at +23 °C (C3 and C4 control samples) started to show up to 20% activity loss of CLT activity already after 1 month of storage at +23 °C, in cases where CLT was stored in the Spray or Gel formulations (Prepl:(Spray) and Prep2:(Gel) samples) at +23 °C for up to 4 months, the activity of CLT surprisingly remained the same as before storage of the formulation.

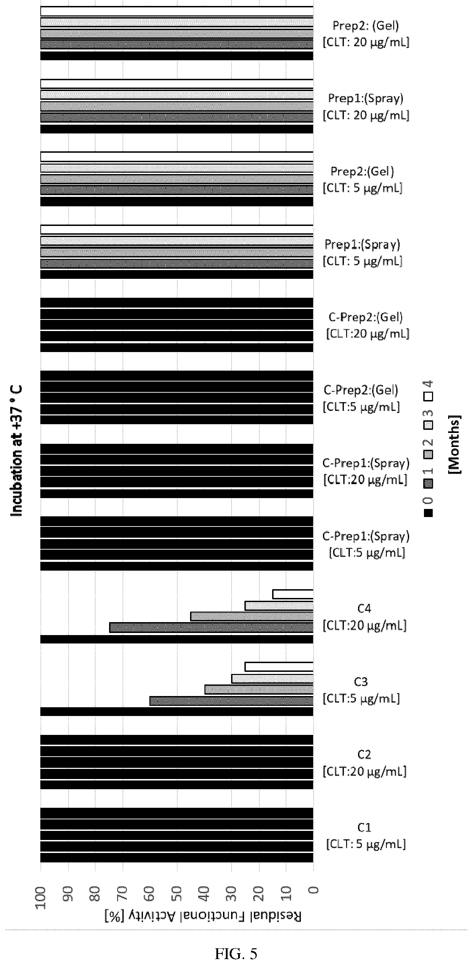

### 3c. Incubation at +37 ° C (FIG. 5)

The samples were formulated and stored using the same principle as in 3a experiment, except that the storage of up to 4 months was performed at +37 °C temperature. The scratch assay and respective calculations for presentation of the results were performed and presented in the same manner as the assays in Example 3a. The results are provided in FIG. 5; SD values were ranging within the range of 5-15%.

As can be seen from the results in Fig. 5, while the control samples that were stored for up to 4 months at +37 °C (C3 and C4 control samples) started to show about 25% (in case of C4) and even about 40% (in case of C3) activity loss of CLT activity already after 1 month of storage at +37 °C, whereas in cases where CLT was stored in the Spray or Gel formulations (Prepl:(Spray) and Prep2:(Gel) samples) at +37 °C for up to 4 months, the activity of CLT surprisingly remained the same as before storage of the formulation.

### Example 4. Functional activity results in vitro

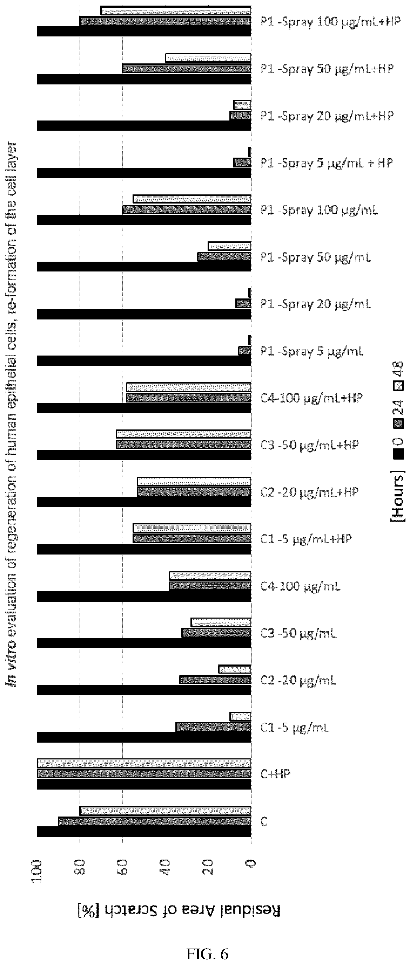

### 4a. Spray- in vitro evaluation of regeneration of human epithelial cells, re-formation of the cell layer (FIG.6)

Human Epidermal keratinocytes HaCAT-KER-CTh (skin) cells were cultured in a serum for cultivation of epithelial cells (Gibcol) free of supplements. After reaching confluence and forming monolayers, scratch was conducted and cell monolayers were exposed to various concentrations of freshly made CLT and CLT based preparations. Hydrogen peroxide (0,0003%) was used as control ("HP"), able to induce toxicity and impair wound healing. Effects were assessed after 24h and 48h at room temperature. The gap of the scratch was evaluated with microscope, the image was processed with filters and was measured using ImageJ software. The percentage of scratch area change during the experimental course was used for generation of healing charts. 100 % value represents area of the fresh scratch at the healing starting point.

C -Layer of human Epidermal keratinocytes cells after scratch treated with phosphate buffer. C+HP-Layer of human Epidermal keratinocytes cells after scratch and constant treatment with hydrogen peroxide with phosphate buffer.

C1 -C4 - Layer of human Epidermal keratinocytes cells after scratch and constant treatment with pure CLT protein with the concentration 5, 20, 50 and 100 µg /mL respectively, formulated in phosphate buffer.

C1 -C4 + HP - Layer of human Epidermal keratinocytes cells after scratch and constant treatment with hydrogen peroxide and pure CLT protein with the concentration 5, 20, 50 and 100 µg /mL respectively, formulated in phosphate buffer. The gap of scratched cells was monitored after 24 and 48 hours, respectively. The spike of hydroxy peroxide was repeated after 24 hours. P1-Prep1 Spray group, final product formulation containing 5, 20, 50 and 100 µg /mL of CLT were applied on human Epidermal keratinocytes after scratch. The gap of scratched cells was monitored after 24 and 48 hours, respectively.

P1-Prep1 Spray + HP group, final product formulation containing 5, 20, 50 and 100 µg /mL of CLT were applied on human epidermal keratinocytes cells which were treated with hydroxy peroxide, after scratch. The gap of scratched cells was monitored after 24 and 48 hours, respectively. The spike of hydroxy peroxide was repeated after 24 hours.

SD values were ranging within the range of 5-15%.

As can be seen from Fig. 6, using various CLT Spray preparations worked well. CLT in Prep1:Spray formulations with 5, 20, 50 µg /mL of CLT provided for much faster re-formation of the cell layer, compared to respective control samples; the effect was observed in both cases, with and without H2O2. Further, in Prepl:Spray formulation cases where CLT was presented at concentrations of 5 and 20 µg /mL, the regeneration was advantageously faster and much more efficient compared with the control CLT formulations in phosphate buffer.

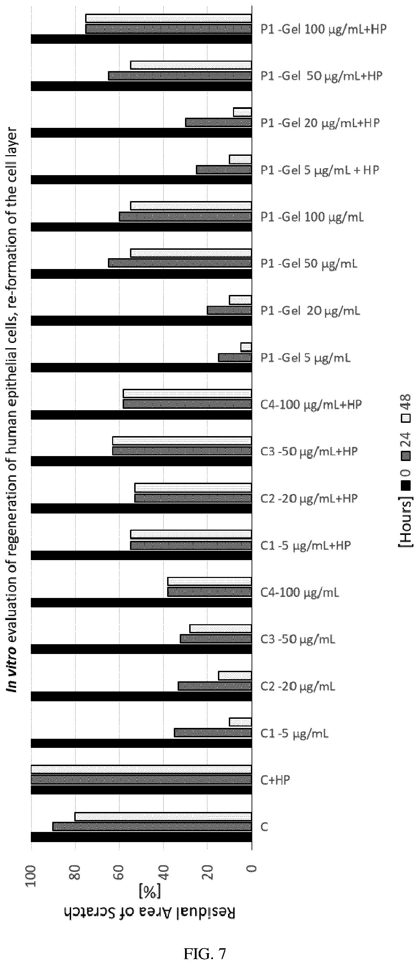

### 4b. GEL-in vitro evaluation of regeneration of human epithelial cells, re-formation of the cell layer (FIG. 7)

The experiments were performed and evaluated in the same manner as in 4a experiment, except that the gel formulations were used.

C -Layer of human Epidermal keratinocytes cells after scratch treated with phosphate buffer. C+HP-Layer of human Epidermal keratinocytes cells after scratch and constant treatment with hydrogen peroxide with phosphate buffer.

C1 -C4 - Layer of human Epidermal keratinocytes cells after scratch and constant treatment with pure CLT protein with the concentration 5, 20, 50 and 100 µg /mL respectively, formulated in phosphate buffer.

C1 -C4 + HP - Layer of human Epidermal keratinocytes cells after scratch and constant treatment with hydrogen peroxide and pure CLT protein with the concentration 5, 20, 50 and 100 µg /mL respectively, formulated in phosphate buffer. The gap of scratched cells was monitored after 24 and 48 hours, respectively. The spike of hydroxy peroxide was repeated after 24 hours.

P1 - Prep2:Gel group, final product formulation containing 5, 20, 50 and 100 µg /mL of CLT were applied on human Epidermal keratinocytes after scratch. The gap of scratched cells was monitored after 24 and 48 hours, respectively.

P1 - Prep2:Gel + HP group, final product formulation containing 5, 20, 50 and 100 µg /mL of CLT were applied on human epidermal keratinocytes cells which were treated with hydroxy peroxide, after scratch. The gap of scratched cells was monitored after 24 and 48 hours, respectively. The spike of hydroxy peroxide was repeated after 24 hours.

SD values were ranging within the range of 5-15%.

As can be seen from Fig. 7, various CLT Gel preparations worked well. Using CLT in Prep2:Gel formulations with 5 and 20 µg /mL of CLT provided for much faster re-formation of the cell layer, compared to respective control samples; the effect was observed in both cases, with and without H2O2.

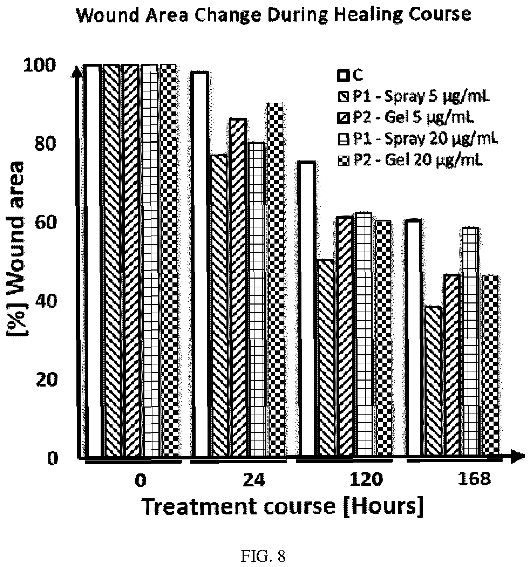

### Example 5. Functional activity results in vivo (FIG. 8)

Surgical wound healing evaluation in vivo using mice strain C57BL/6J. Four groups of mice were used for systematic evaluation of wound healing effects of two bio-formulations. Mice groups: Control (C), wounded mice treated using conventional wound management and treatment flow (Wound Cleaning, disinfection and application of sterile dressing), P1 and P2 after injurie treatment with freshly made bio-formulations Prep1 [P1- Spray: 5 µg/mL and 20 µg/mL ] and Prep2 [P2 Gel: 5 µg/mL and 20 µg/mL], respectively by following protocol ((Wound Cleaning, disinfection, application of Prep1 [5 µg/mL and 20 µg/mL ] and Prep2 [5 µg/mL and 20 µg/mL ] and application of sterile dressing). The injury was created by excision as described by Fischer KS, et al. Bio Protoc. 2023 Feb 5;13(3):e4606. The treatment using Prep 1 and Prep 2 was continued and monitored for 7 days or 168 hours at room temperature. The healing effects were evaluated by measuring wound area with ImageJ (Fiji). The percentage of wound area change during the healing course was used for generation of healing charts. 100 % value represents area of the fresh wound at the healing starting point. SD values were ranging within the range of 5-15%. Experimental data (FIG. 8) revealed that reduction of wound area was achieved significantly faster in the mice groups of P1-Spray (5 µg/mL) and P2 -Gel (5 µg/mL) which were treated with Prep1 and PreP2, respectively. Both preparations with 20 µg/mL also exhibited significant stimulating effects of reduction of surgical wound area.

## Claims

1. A stabilized pharmaceutical composition comprising calreticulin protein or a functional fragment or derivative thereof and one or more of pharmaceutically acceptable polymeric materials that form hydrogel for use in medical treatment.

2. The stabilized pharmaceutical composition for use according to claim 1 wherein the pharmaceutical composition is formulated for topical administration and wherein the medical treatment is treatment of chronic and acute wounds.

3. The stabilized pharmaceutical composition for use according to claims 1 or 2, wherein the calreticulin is human calreticulin, preferably wherein the calreticulin has the amino acid sequence of SEQ ID NO: 1 or an amino acid sequence having at least 85% sequence identity to the amino acid sequence of SEQ ID NO: 1.

4. The stabilized pharmaceutical composition for use according to any preceding claims wherein the calreticulin is recombinant, preferably wherein the calreticulin is obtained by recombinant expression in yeast cells, such as *Saccharomyces cerevisiae* or *Pichia pastoris.*

5. The stabilized pharmaceutical composition for use according to any preceding claims, wherein the calreticulin is at a concentration of about from 1 µg/mL to 10 mg/mL, preferably in the range from 5 µg/mL to 1 mg/mL, more preferably in the range between about from 5 µg/mL to 500 µg/mL.

6. The stabilized pharmaceutical composition for use according to any preceding claims, wherein the calreticulin is at a concentration from about 5 µg/mL to 100 µg/mL, preferably wherein calreticulin is at a concentration of 5 µg/mL, 20 µg/mL or 50 µg/mL.

7. The stabilized pharmaceutical composition for use according to any preceding claims, wherein the pharmaceutical composition is formulated as liquid hydrogel spray or a gel for administration to body surfaces.

8. The stabilized pharmaceutical composition for use according to any preceding claims, wherein the pharmaceutically acceptable polymeric materials are selected from carbomer, hydroxyethyl cellulose, hydroxypropyl methyl cellulose and xanthan gum, preferably wherein the pharmaceutically acceptable polymeric materials are selected from hydroxyethyl cellulose and hydroxypropyl methyl cellulose.

9. The stabilized pharmaceutical composition for use according to any one of the preceding claims, wherein the pharmaceutically acceptable polymeric materials are at concentrations in the range from about 0.5% to 10% w/v of the composition.

10. The stabilized pharmaceutical composition for use according to any preceding claims, wherein the pharmaceutically acceptable polymeric material is:
a) hydroxyethyl cellulose at a concentration from 0.1% (w/v) to 4% (w/v), preferably from 0.5% (w/v) to 2% (w/v), more preferably at a concentration of 0.5% (w/v), or
b) hydroxypropyl methyl cellulose at a concentration from 1% (w/v) to 10% (w/v), preferably from 4% (w/v) to 10% (w/v), more preferably at a concentration of 4% (w/v).

11. The stabilized pharmaceutical composition for use according to any one of the preceding claims, wherein the pharmaceutical composition further comprises serum albumin protein at concentrations in the range from about 1 mg /mL to 10 mg /mL.

12. The stabilized pharmaceutical composition for use according to claim 11, wherein serum albumin protein is selected from bovine serum albumin protein and human serum albumin protein.

13. The stabilized pharmaceutical composition for use according to any one of the preceding claims, wherein the pharmaceutical composition further comprises preservatives, such as potassium sorbate and/or sodium benzoate.

14. The stabilized pharmaceutical composition for use according to any one of the preceding claims, wherein pharmaceutical composition further comprises biologically active agents selected from the group consisting of growth factors, antibiotics, anti-inflammatories, analgesics, blood coagulants, and enzymes.

15. The stabilized pharmaceutical composition for use according to any one of the preceding claims, wherein the pharmaceutical composition is stored at temperatures up to 37 °C, preferably 23 °C.
